# EUROPÄISCHE PATENTSCHRIFT

(11) **EP 2 537 463 B1**
(45) Veröffentlichungstag und Bekanntmachung des Hinweises auf die Patenterteilung: **28.10.2015**
(21) Anmeldenummer: 12167276.0
(22) Anmeldetag: 09.05.2012
(51) Int. Cl.: A61B 5/042, A61N 1/05, A61M 39/02

(54) **Medizinisches Sensorsystem**
Medical Sensor System
Système de détection médical

(30) Priorität: 24.06.2011 US 201161500622 P
(43) Veröffentlichungstag der Anmeldung: 26.12.2012
(73) Patentinhaber: BIOTRONIK SE & Co. KG, 12359 Berlin (DE)
(72) Erfinder: Dörr, Thomas, 12437 Berlin (DE)
(74) Vertreter: Galander, Marcus

(56) Entgegenhaltungen:
- US-A1- 2005 182 455
- US-A1- 2009 085 768
- US-A1- 2010 152 812
- US-B1- 7 917 222
- US-B2- 6 643 552

## Beschreibung

Die Erfindung liegt auf dem Gebiet der Medizintechnik und betrifft ein medizinisches Sensorsystem mit einer im menschlichen oder tierischen Körper implantierbaren Sensorleitung mit einem oder mehreren Sensoren zur Erfassung biologischer Messgrößen.

Die Kontraktion des Herzens basiert auf einer elektrischen Erregung, die im Normalfall im Sinusknoten erzeugt und vom herzeigenen Reizleitungssystem auf die Herzmuskelzellen übertragen wird. Die dabei auftretenden Spannungsänderungen können mittels Elektroden an der Körperoberfläche gemessen werden, wobei aus dem zeitlichen Verlauf (EKG = Elektrokardiogramm) Aussagen über Zustand und Erkrankungen des Herzens getroffen werden können. So können durch das Elektrokardiogramm beispielsweise Störungen im Herzrhythmus, insbesondere Tachy- und Bradykardien, sowie ventrikuläre Extrasystolen erkannt werden.

Nun treten Herzrhythmusstörungen oftmals nicht während einer Untersuchung in einer Arztpraxis oder im Krankenhaus auf, sondern in bestimmten Situationen, beispielsweise während körperlicher Belastung oder in der Nacht. Es ist eine gängige Praxis, Patienten einen tragbaren EKG-Rekorder anzulegen, mit dem ein so genanntes Langzeit-EKG für einen Zeitraum von beispielsweise 24 Stunden oder länger aufgezeichnet werden kann. Dabei werden dem Patienten mit dem EKG-Rekorder verbundene Elektroden auf die Brust geklebt und die elektrischen Signale aufgezeichnet. Für den Patienten ist dies mit Unannehmlichkeiten verbunden, da durch die aufgeklebten Elektroden die Körperpflege beeinträchtigt ist und der EKG-Rekorder meist sichtbar am Körper getragen wird. Zudem können die aufgeklebten Elektroden leicht abgehen, so dass für eine längere Beobachtung der elektrischen Signale des Herzens, beispielsweise für einen Zeitraum über mehrere Wochen, Monate oder Jahre ein solcher tragbarer EKG-Rekorder nicht geeignet ist.

Aus der medizinischen Praxis bekannt sind im Körper des Patienten implantierbare EKG-Rekorder. Hierbei wird dem zu überwachenden Patienten ein EKG-Rekorder in Form eines vollständig gekapselten Implantats in einer Implantattasche subkutan oder submuskulär nahe dem Herzen implantiert. Das Implantat ist typischer Weise mit einer Telemetrieschnittstelle versehen, durch die Informationen abgefragt werden können. Nachteilig bei implantierbaren EKG-Rekordern ist die Tatsache, dass die Implantation relativ aufwändig ist und die Implantattasche eröffnet werden muss, sobald die Diagnostik abgeschlossen oder die Batterie des Implantats erschöpft ist. Dies ist für den Patienten beschwerlich, hinterlässt eine relativ große Narbe und birgt durch die Implantattasche ein nicht unerhebliches Infektionsrisiko. Ein weiterer Nachteil sind die relativ großen Abmessungen des Implantats, die sich in wesentlicher Weise aus der von der Einsatzzeit abhängigen Batteriegröße, der Größe der Antenne für die Telemetrieschnittstelle und der biokompatiblen Kapselung des Implantats bestimmen. In der Praxis sind Implantate daher in der Regel relativ groß. Dies hat zur Folge, dass die Implantate nicht primär hinsichtlich einer optimalen EKG-Ableitung angepasst und in Anbetracht anatomischer Gegebenheiten oftmals nicht an einem für die EKG-Ableitung günstigsten Ort platziert werden können, worunter die Qualität der Diagnostik leiden kann. Ein weiterer Nachteil sind die relativ hohen Kosten eines solchen implantierbaren EKG-Rekorders, da alle Komponenten den Ansprüchen eines implantierbaren Medizinprodukts genügen müssen, die Batterie entsprechend groß ausgelegt werden muss, um ein vorzeitiges Entfernen zu vermeiden, und eine vergleichsweise teure Telemetrieschnittstelle vorzusehen ist. Darüber hinaus gibt es insbesondere bei jüngeren Patienten Akzeptanzprobleme, da ein solches Implantat aufträgt und die Implantation wie bereits erwähnt eine relativ große Narbe hinterlässt. Zudem kann das Implantat für bestimmte Diagnose- und Therapieverfahren (z.B. kernspintomographische Untersuchung) eine Kontraindikation darstellen.

Aus der US210/152812 ist ein Neurostimulator bekannt, der ein externes Gehäuse und einen implantierten Teil aufweist. Die US6,643,552 beschreibt ein implantierbares Gerät, basieren auf einem Substrat aus "Liquid Crystal Polymers". Die US2009/085768, die US2005/182455 und die US791722 beziehen sich auf Vorrichtungen teilweise implantiert oder implantierbar sind.

Demgegenüber besteht die Aufgabe der vorliegenden Erfindung darin, die genannten Nachteile von im Stand der Technik bekannten implantierbaren oder tragbaren EKG-Rekordem zu vermeiden. Diese und weitere Aufgaben werden nach dem Vorschlag der Erfindung durch ein medizinisches Sensorsystem mit den Merkmalen des unabhängigen Patentanspruchs gelöst. Vorteilhafte Ausgestaltungen der Erfindung sind durch die Merkmale der Unteransprüche angegeben.

Erfindungsgemäß ist ein Sensorsystem zur medizinischen Anwendung gezeigt. Das Seinsorsystem umfasst mindestens eine im menschlichen oder tierischen Körper implantierbare Sensorleitung mit zumindest einem Sensor zum Erfassen einer biologischen Messgröße. Wie hier verwendet, bezieht sich der Ausdruck "implantierbar" auf einen dauerhaften Verbleib im Körper, beispielsweise während eines Zeitraums von einigen Wochen, einigen Monaten oder einigen Jahren. Umfasst sein soll aber auch ein nur kurzzeitiger, vorübergehender Verbleib im Körper eines Patienten, beispielsweise während einer Untersuchung. Die Sensorleitung des medizinischen Sensorsystems ist vorzugsweise für eine subkutane Implantation vorgesehen.

Die implantierbare Sensorleitung umfasst erfindungsgemäß zwei Endabschnitte, die dazu ausgebildet sind, durch zwei künstliche Körperöffnungen an die Körperoberfläche geführt zu werden, so dass die beiden Endabschnitte an der Körperoberfläche kontaktierbar sind. Dabei befindet sich der zumindest ein Sensor in einem zwischen den beiden Endabschnitten angeordneten Zwischenabschnitt der implantierten Sensorleitung. Der Sensor kann insbesondere als biophysikalischer oder biochemischer Sensor ausgebildet sein. Ferner kann es sich bei dem Sensor um ein aktiven Sensor, der zur Aufnahme einer Messgröße und zum Erzeugen von Sensorsignalen elektrische Energie benötigt, oder um einen passiven Sensor, welcher auch ohne Zuführen von elektrischer Energie eine Messgröße aufnehmen und Sensorsignale abgeben kann, ausgebildet sein.

Das Sensorsystem umfasst weiterhin wenigstens ein auf der Körperoberfläche angeordnetes Fixierelement, welches mit den durch die Körperöffnungen geführten Endabschnitten der Sensorleitung zur (Lage-)Fixierung derselben verbindbar bzw. verbunden ist. Die beiden Endabschnitte und das mindestens eine Fixierelement sind zu diesem Zweck mit geeigneten Verbindungen, beispielsweise Schraub-, Klemm-, Steck-, Rast- oder Clipsverbindungen, versehen.

In dem erfindungsgemäßen Sensorsystem weist das zumindest eine Fixierelement eine Einrichtung zum Prozessieren von Signalen des Sensors sowie eine Energiequelle zur Versorgung des Sensorsystems mit elektrischer Energie auf.

Die Einrichtung zum Prozessieren von Sensorsignalen kann in vielfältiger Weise ausgebildet sein, wobei der Begriff "Prozessieren von Sensorsignalen" breit zu verstehen ist. So kann die Einrichtung insbesondere dazu dienen, die Sensorsignale zu speichern, zu verarbeiten, auszuwerten, bereitzustellen und/oder zu übertragen. Die Einrichtung zum Prozessieren von Sensorsignalen ist zu diesem Zweck mit dem mindestens einen Sensor elektrisch verbunden, wenn die Sensorleitung mit dem mindestens einen Fixierelement verbunden ist. Insbesondere ist die Sensorleitung dazu ausgebildet, die vom zumindest einen Sensor abgegebenen Sensorsignale an zumindest einen der beiden Endabschnitte, insbesondere an beide Endabschnitte zu leiten, so dass bei der Verbindung der Sensorleitung mit dem mindestens einen Fixierelement die Sensorsignale in einfacher Weise an die Einrichtung zum Prozessieren von Sensorsignalen übertragen werden können.

Die Energiequelle kann zur Energiespeisung der Einrichtung zum Prozessieren von Sensorsignalen und/oder des Sensors dienen. Zu diesem Zweck ist die Energiequelle mit der Einrichtung zum Prozessieren von Sensorsignalen und/oder dem Sensor elektrisch verbindbar bzw. verbunden. Typischer Weise verfügt die Einrichtung zum Prozessieren von Sensorsignalen über einen Mikroprozessor und ist programmtechnisch zum Prozessieren der Sensorsignale in geeigneter Weise eingerichtet.

Das erfindungsgemäße Sensorsystem ermöglicht somit in vorteilhafter Weise, dass lediglich die Sensorleitung mit dem zumindest einen Sensor im Körper, insbesondere subkutan, implantiert wird, wobei die Einrichtung zum Prozessieren von Sensorsignalen und die Energiequelle zum Betreiben des Systems auf der Körperoberfläche angeordnet werden, so dass die Sensorleitung entsprechend klein dimensioniert werden kann. Die Sensorleitung kann dadurch sehr einfach und mit einem stark verringerten Risiko von Komplikationen implantiert und insbesondere temporär explantiert werden. Eine Implantation erfolgt durch die beiden Körperöffnungen für die Endabschnitte, welche zumindest annähernd dem Durchmesser der Sensorleitung entsprechen, so dass nach Entfernen des Implantats keine oder nur sehr kleine Narben verbleiben. Zudem können die Sensorleitung und das auf der Körperoberfläche angeordnete wenigstens eine Fixierelement in kosmetischer Hinsicht sehr ansprechend gestaltet werden. Das wenigstens eine Fixierelement ermöglicht eine zuverlässige und sichere Fixierung der Sensorleitung im Körper des Patienten. Da das zumindest eine Fixierelement nicht den speziellen Anforderungen einer implantierbaren medizinischen Komponente genügen muss, kann das Sensorsystem einfach und kostengünstig realisiert werden.

Allgemein kann das mindestens eine Fixierelement des Sensorsystems in beliebiger Weise ausgebildet sein, solange eine Fixierung der implantierten Sensorleitung und eine Anordnung der Einrichtung zum Prozessieren von Sensorsignalen sowie der Energiequelle gewährleistet sind. Bei einer vorteilhaften Ausgestaltung des erfindungsgemäßen Sensorsystems ist das wenigstens eine Fixierelement zur Anpassung an die Kontur der Körperoberfläche flexibel ausgebildet, wodurch eine besonders effektive Fixierung der Sensorleitung erreicht werden. Zudem kann dadurch der Tragekomfort für den Patienten und die ästhetische Erscheinung des Sensorsystems verbessert werden, so dass das Sensorsystem eine bessere Akzeptanz beim Patienten erlangt.

Das mindestens eine Fixierelement ist mit den beiden durch die Körperöffnungen geführten Endabschnitte der Sensorleitung verbindbar bzw. verbunden. Von Vorteil ist es, wenn das mindestens eine Fixierelement mit den beiden Endabschnitten der Sensorleitung abnehmbar verbindbar ist, so dass es von den Endabschnitten getrennt und mit diesen wieder verbunden werden kann. Die Möglichkeit einer wiederverbindbaren Befestigung des mindestens einen Fixierelements an den beiden Endabschnitten ermöglicht in besonders vorteilhafter Weise einen Austausch und/oder eine Wartung des zumindest einen Fixierelements, ohne die Notwendigkeit die implantierte Sensorleitung entfernen zu müssen. Insbesondere kann die Energiequelle (Batterie) des Sensorsystems in einfacher Weise nachgeladen und/oder ausgetauscht werden, wodurch insbesondere ermöglicht ist, dass relativ preiswerte (z.B. kleinere) Energiequellen eingesetzt werden. Ein Abnehmen des zumindest einen Fixierelements kann aber auch dazu benutzt werden, Daten aus der Einrichtung zum Prozessieren von Sensorsignalen auszulesen, so dass auch auf eine relativ kostenintensive Telemetrieschnittstelle zum Übertragen von Sensorsignalen oder hierauf basierender Daten verzichtet werden kann.

In dem erfindungsgemäßen Sensorsystem kann die implantierbare Sensorleitung starr oder flexibel ausgebildet sein, wobei es für das Aufnehmen von Messgrößen von Vorteil sein kann, wenn die Sensorleitung flexibel ist, beispielsweise um den wenigstens einen Sensor an einem für die Signalableitung anatomisch besonders geeigneten Ort zu platzieren. Zudem ist es von Vorteil, wenn die Endabschnitte der Sensorleitung flexibel und/oder gekrümmt bzw. gebogen sind, so dass diese in einfacher Weise durch die beiden Körperöffnungen an die Körperoberfläche geführt werden können.

Bei einer weiteren vorteilhaften Ausgestaltung des erfindungsgemäßen Sensorsystems weist die Sensorleitung einen konstanten (unveränderten) Leitungsdurchmesser und/oder keine Sprünge im Leitungsdurchmesser auf, wodurch erreicht werden kann, dass die Sensorleitung in besonders einfacher Weise entfernt und wieder implantiert werden kann. Insbesondere könnte dies gegebenenfalls auch vom Patienten selber ausgeführt werden.

Wie dargestellt, verfügt das erfindungsgemäße Sensorsystem über mindestens ein Fixierelement zur Fixierung der implantierten Sensorleitung im Körper des Patienten. Erfindungsgemäß ist lediglich ein einziges Fixierelement zur Fixierung der implantierten Sensorleitung vorgesehen, das mit den beiden Endabschnitten der Sensorleitung verbindbar bzw. verbunden ist. Das einzige Fixierelement kann insbesondere zur Anpassung an die Kontur der Körperoberfläche flexibel ausgebildet sein, um eine zuverlässige und sichere Fixierung der Sensorleitung zu erreichen. Dabei ist das einzige Fixierelement mit einem der beiden Endabschnitte permanent verbunden, wobei es relativ zur Sensorleitung bewegbar ist, und mit dem anderen Endabschnitt der Sensorleitung verbindbar sein. Zu diesem Zweck verfügt das Sensorsystem beispielsweise über einen Träger aus einem flexiblen Material verfügt, wobei die Einrichtung zum Prozessieren von Sensorsignalen und die Energiequelle zur Versorgung des Sensorsystems mit elektrischer Energie am Träger befestigt sind und wobei die Sensorleitung in Form einer versteiften Struktur des Trägers ausgeformt ist. Insbesondere kann der Träger für das Aufbringen gedruckter Schaltungen geeignet ausgebildet sein, um elektrische Verbindungen zwischen den verschiedenen elektrischen Komponenten in einfacher Weise realisieren zu können. Diese Ausgestaltung ermöglicht einerseits einen besonders einfachen Aufbau des Sensorsystems, der andererseits eine sehr schnelle Implantation der Sensorleitung ermöglicht. Hierfür muss lediglich die schon an einem Endabschnitt mit dem Fixierelement verbundene Sensorleitung in eine für die Implantation geeignete Stellung gebracht werden, was durch die flexible Anbindung an das Fixierelement möglich ist, und anschließend nach erfolgter Implantation der Sensorleitung der andere Endabschnitt der Sensorleitung mit dem Fixierelement verbunden werden.

Bei einem alternativen Sensorsystem sind zwei Fixierelemente zur Fixierung der Sensorleitung vorgesehen, die jeweils mit einem der beiden Endabschnitte der Sensorleitung verbindbar bzw. verbunden sind. Auch diese Ausgestaltung ermöglicht eine zuverlässige und sichere Fixierung der im Körper implantierten Sensorleitung, gegebenenfalls auch bei starren Fixierelementen, die im Vergleich zur vorigen Ausgestaltung des Sensorsystems mit einem einzigen Fixierelement kleiner ausgebildet sein können. Dabei kann das Sensorsystem insbesondere so ausgebildet sein, dass das eine Fixierelement die Einrichtung zum Prozessieren von Sensorsignalen und das andere Fixierelement die Energiequelle aufweist, so dass die Einrichtung zum Prozessieren von Sensorsignalen und die Energiequelle unabhängig voneinander ausgetauscht und/oder gewartet werden können. Beispielsweise können Daten von der Einrichtung zum Prozessieren von Sensorsignalen ausgelesen werden, ohne gleichzeitig die Energieversorgung des Systems zu entfernen. Alternativ hierzu kann das Sensorsystem auch so ausgebildet sein, dass nur eines der beiden Fixierelemente die Einrichtung zum Prozessieren von Sensorsignalen und die Energiequelle aufweist, wohingegen das andere Fixierelement lediglich eine fixierende bzw. abschließende Funktion hat.

Wie bereits ausgeführt wurde, können die Sensorsignale beispielsweise dann ausgelesen werden, wenn das zumindest eine Fixierelement von der Sensorleitung entfernt wird. Gleichwohl kann es von Vorteil sein, wenn die Einrichtung zum Prozessieren von Signalen des Sensors über eine Telemetrieeinheit mit einer Telemetrieschnittstelle zur drahtlosen Übertragung von Sensorsignalen und/oder hierauf basierender Daten an eine körperferne elektronische Einrichtung verfügt bzw. damit datentechnisch gekoppelt ist.

Bei einer weiteren vorteilhaften Ausgestaltung des erfindungsgemäßen Sensorsystems weist die Sensorleitung mindestens ein Wirkstoffdepot zur Abgabe eines oder mehrerer Wirkstoffe an den Körper auf. Insbesondere können hierdurch infektionshemmende oder die Epithelialisierung fördernde Wirkstoffe in den Körper eingeschleust werden.

Bei einer weiteren vorteilhaften Ausgestaltung des erfindungsgemäßen Sensorsystems weist es eine Sicherungseinrichtung zum Lösen der Verbindung zwischen dem mindestens einen Fixierelement und den beiden Endabschnitten der Sensorleitung auf. Durch diese Maßnahme kann ein versehentliches und/oder nicht autorisiertes Lösen der Verbindung verhindert werden.

Bei einer weiteren vorteilhaften Ausgestaltung des erfindungsgemäßen Sensorsystems ist die Sensorleitung in Form einer bipolaren Elektrodenleitung mit zumindest einer Elektrode ausgebildet, wobei die Elektrode zum Erfassen elektrischer Signale im Körper des Patienten, insbesondere elektrischer Spannungen im Zusammenhang mit Herzkontraktionen, dient. Die Elektrode kann insbesondere als elektrisch leitender Oberflächenbereich zur Aufnahme elektrischer Signale der Elektrodenleitung ausgebildet sein, wobei die Elektrode grundsätzlich aber auch zur Abgabe elektrischer Impulse, d.h. zur Übertragung von elektrischer Energie an die Umgebung dienen kann. Eine andere übliche Bezeichnung für Elektrode ist "Pol". Die zumindest eine Elektrode der Elektrodenleitung ist mit einer elektrischen Zuleitung verbunden, durch welche sie an einem oder beiden Endabschnitten mit der Einrichtung zum Prozessieren von Sensorsignalen elektrisch verbindbar bzw. verbunden ist. Die Oberfläche der Elektrodenleitung ist mit Ausnahme der zumindest einen Elektrode in der Regel elektrisch isoliert. Das Sensorsystem kann insbesondere als EKG-Rekorder ausgebildet sein.

Es versteht sich, dass die verschiedenen Ausgestaltungen des erfindungsgemäßen Sensorsystems einzeln oder in beliebigen Kombinationen realisiert sein können. Insbesondere sind die vorstehend genannten und nachstehend zu erläuternden Merkmale nicht nur in den angegebenen Kombinationen, sondern auch in anderen Kombinationen oder in Alleinstellung einsetzbar, ohne den Rahmen der vorliegenden Erfindung zu verlassen.

Die Erfindung wird nun anhand von Ausführungsbeispielen näher erläutert, wobei Bezug auf die beigefügten Zeichnungen genommen wird. Gleiche bzw. gleich wirkende Elemente sind mit denselben Bezugszahlen bezeichnet. Es zeigen:
- Fig. 1: eine schematische Darstellung eines Patienten zur Veranschaulichung einer implantierten Sensorleitung;
- Fig. 2: eine schematische Darstellung eines Sensorsystems;
- Fig. 3: eine schematische Darstellung des im Körper eines Patienten implantierten Sensorsystems von Fig. 2;
- Fig. 4: eine schematische Darstellung eines weiteren Sensorsystems;
- Fig. 5: eine schematische Darstellung eines Ausführungsbeispiels des erfindungsgemäßen Sensorsystems;
- Fig. 6A-6B: weitere schematische Darstellungen des Sensorsystems von Fig. 5;
- Fig. 7: eine schematische Darstellung des im Körper eines Patienten implantierten Sensorsystems von Fig. 5;
- Fig. 8: eine schematische Darstellung eines im Körper eines Patienten implantierten herkömmlichen EKG-Rekorders.

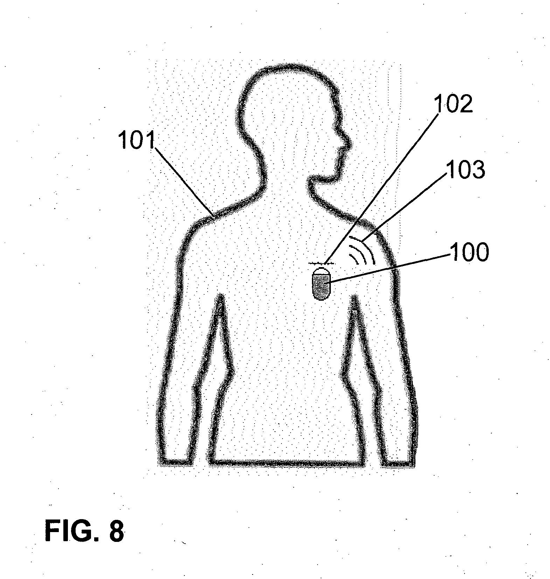

Sei zunächst Fig. 8 betrachtet, worin ein im Körper eines Patienten implantierter, vollständig gekapselter EKG-Rekorder 100 gemäß dem Stand der Technik veranschaulicht ist. Demnach ist der EKG-Rekorder 100 nahe dem Herzen eines Patienten 101 in einer Implantattasche 102 implantiert. Der EKG-Rekorder 100 verfügt über eine nicht näher dargestellte Telemetrieeinrichtung mit einer Telemetrieschnittstelle, durch welche Daten 103 durch Nah- oder Fernfeldtelemetrie aus dem Implantat abgefragt werden können. Die Nachteile eines solchen Implantats wurden eingangs bereits eingehend erläutert.

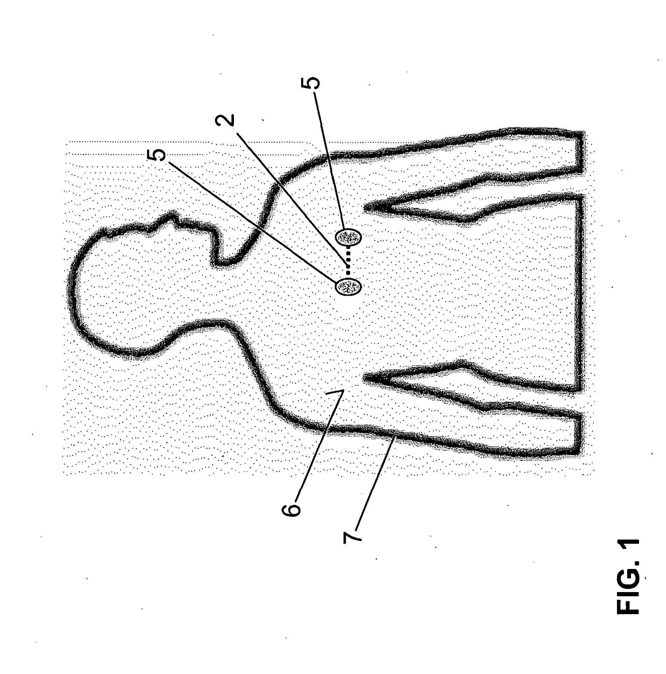

In den Figuren 1 bis 3 ist ein erstes Sensorsystem veranschaulicht, welches insgesamt mit der Bezugszahl 1 bezeichnet ist. Das Sensorsystem 1 umfasst eine zur subkutanen Implantation vorgesehene Sensorleitung, die hier beispielsweise als bipolare (EKG-)Elektrodenleitung 2 ausgeführt ist. Die Elektrodenleitung 2 kann zumindest gedanklich in zwei Endabschnitte 3, 3' und einem zwischen den beiden Endabschnitten 3,3' befindlichen Zwischenabschnitt 4 unterteilt werden. Die Unterteilung ergibt sich hier beispielsweise durch die Form der Elektrodenleitung 2, wobei die beiden geraden Endabschnitte 3, 3' gegenüber dem geraden Zwischenabschnitt 4 stumpfwinklig angestellt sind, aber gleichermaßen auch einen gekrümmten bzw. gebogenen Verlauf aufweisen könnten.

Wie in Fig. 1 veranschaulicht, kann die Elektrodenleitung 2 nahe dem Herzen eines Patienten 7 subkutan implantiert werden. Bei implantierter Elektrodenleitung 2 sind die beiden Endabschnitte 3, 3' jeweils durch eine künstliche Körperöffnung bzw. Perforationsstelle 5 verlegt, so dass die Elektrodenleitung 2 an ihren beiden Enden an die Körperoberfläche 6 des Patienten 7 gelangt. Somit hat der Zwischenabschnitt 4 eine subkutane Lage, wohingegen sich die beiden Endabschnitte 3, 3' jeweils von einer subkutanen Lage bis zur Körperoberfläche 6 erstrecken. In funktioneller Hinsicht können die beiden Endabschnitte 3, 3' vom Zwischenabschnitt 4 somit auch dahingehend unterschieden werden, dass die Endabschnitte 3 im Unterschied zum Zwischenabschnitt 4 dazu dienen, die Sensorleitung 2 durch die beiden Perforationsstellen 5 an die Körperoberfläche 6 zu führen.

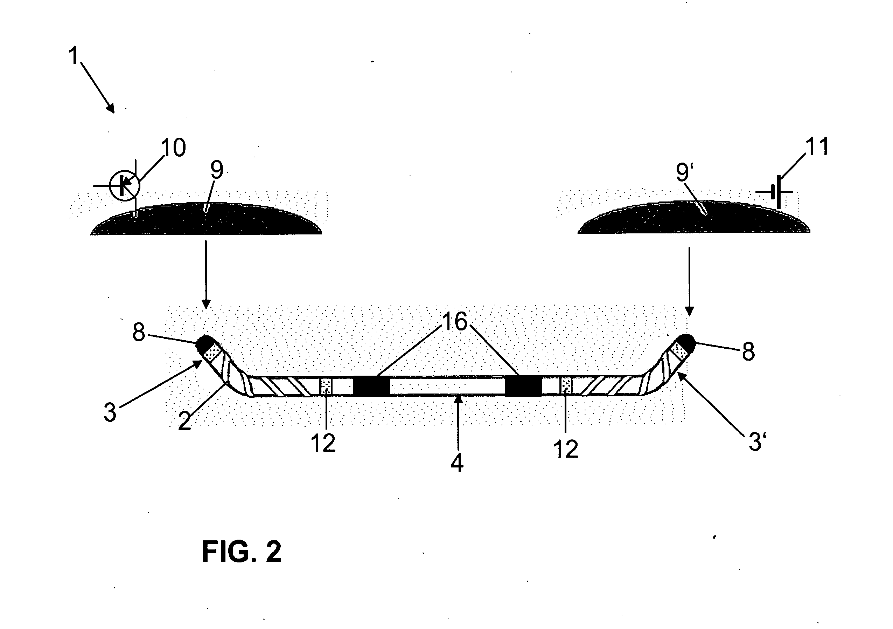

Die Elektrodenleitung 2 kann flexibel oder starr ausgebildet sein. Insbesondere sind die beiden Endabschnitte 3, 3' flexibel ausgebildet und/oder gekrümmt bzw. gebogen, um eine Anpassung an die Körperform im Bereich der Perforationsstellen 5 zu ermöglichen. Wie in Fig. 2 gezeigt, hat die Elektrodenleitung 2 einen symmetrischen Aufbau bezüglich einer den Zwischenabschnitt 4 mittig querenden Symmetrieebene.

Die Elektrodenleitung 2 weist im Zwischenabschnitt 4, d.h. zwischen den beiden Endabschnitten 3, 3', zwei als Sensoren für elektrische Signale dienende Elektroden bzw. elektrische Pole 16 auf, die beiderseits der Symmetrieebene angeordnet sind. Die beiden Pole 16 dienen hier zur Ableitung von EKG-Signalen. Obgleich zwei Pole 16 gezeigt sind, versteht es sich, dass eine größere oder geringe Anzahl von Polen 16 vorgesehen sein kann. Die Pole 16 sind jeweils als elektrisch leitender Oberflächenbereich der Elektrodenleitung 2 ausgebildet, wohingegen die übrige Oberfläche der Elektrodenleitung 2 elektrisch isoliert ist. Über eine elektrische Zuleitung (nicht gezeigt) sind die beiden Pole 16 elektrisch kontaktiert, wobei die EKG-Signale durch die Zuleitung an die beiden Endabschnitte 3, 3' übertragen werden.

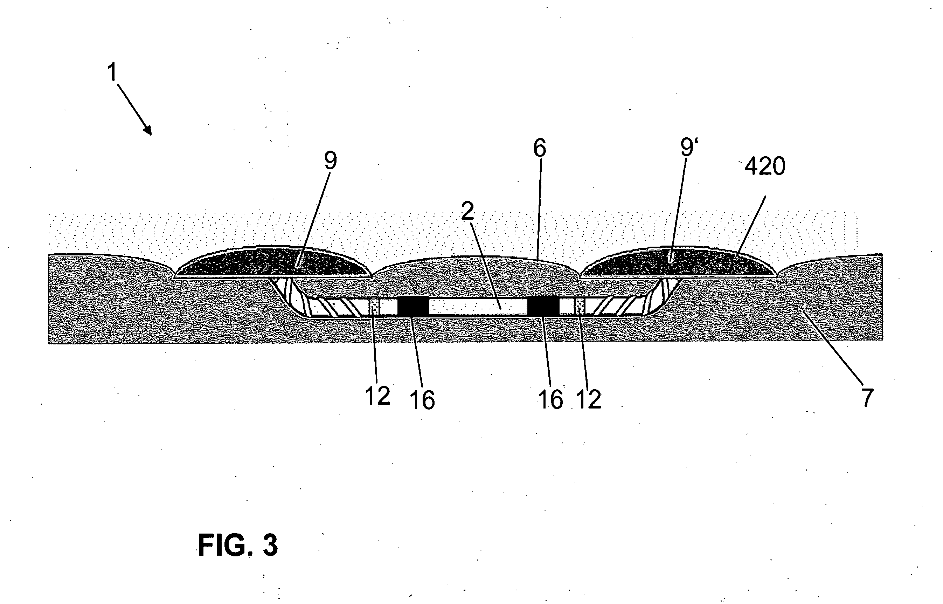

An den beiden Endabschnitten 3, 3' der Elektrodenleitung 2 ist jeweils eine Verschluss- bzw. Kontakteinrichtung 8 angebracht, durch welche die beiden Endkappen 9, 9' an der implantierten Elektrodenleitung 2 abnehmbar (wiederverbindbar) befestigt werden können. Die beiden Endkappen 9, 9' verfügen hier beispielsweise über starres Gehäuse mit einer flachen bzw. ebenen Unterseite, die auf der Körperoberfläche 6 zu liegen kommt. Wie in Fig. 3 veranschaulicht, liegen die Endkappen 9, 9' aufgrund ihrer relativ kleinen Größe der nachgiebigen Körperoberfläche 6 zumindest annähernd passgenau auf. Durch die beiden Endkappen 9, 9' wird die implantierte Elektrodenleitung 2 in ihrer Lage im Körper des Patienten 7 fixiert. Die beiden Verschluss- und Kontakteinrichtungen 8 zur Verbindung der Endkappen 9, 9' können beispielsweise als leicht zu lösende und wiederverbindbare Schraub-, Klemm-, Steck-, Rast- oder Clipsverbindungen ausgebildet sein. Vorteilhaft sind die Verschluss- und Kontakteinrichtungen 8 so ausgebildet, dass deren Lösen ein spezielles Werkzeug erfordert, so dass ein Lösen der Verbindungen nur durch eine autorisierte Person, insbesondere medizinisches Personal, erfolgen kann. Zudem kann auf diese Weise ein Lösen der Verbindung durch den Patienten 7 verhindert werden.

Neben einer Lagefixierung der Elektrodenleitung 2 haben die beiden Endkappen 9, 9' weitere Funktionen. So ist in die eine Endkappe 9 eine mikroprozessorbasierte Diagnoseeinheit 10 zur Prozessierung von EKG-Signalen integriert, in Fig. 3 angedeutet durch ein Transistorsymbol. Die Diagnoseeinheit 10 ist über die genannte Zuleitung mit den beiden Polen 16 elektrisch verbunden, sobald die Endkappe 9 mit dem zugehörigen Endabschnitt 3 der Elektrodenleitung 2 verbunden wird, so dass die EKG-Signale an die Diagnoseeinheit 10 abgeleitet werden können. In der Diagnoseeinheit 10 können die EKG-Signale gespeichert und verarbeitet, insbesondere analysiert werden, wobei Analyseergebnisse widerspiegelnde Daten auf Basis der EKG-Signale generierbar sind. So verfügt die Diagnoseeinheit 10 insbesondere über einen elektronischen Datenspeicher (nicht gezeigt) zur Speicherung von EKG-Signalen oder hierauf basierender Daten, welcher beispielsweise als wechselbare Speicherkarte (z.B. SD-Chipkarte) ausgebildet sein kann.

In die andere Endkappe 9' ist eine in Form einer Batterie 11 ausgebildet Energiequelle integriert, in Fig. 3 angedeutet durch ein Plus-/Minuspolsymbol. Durch die Batterie 11 wird die Diagnoseeinheit 10 zur Prozessierung der EKG-Signale mit elektrischer Energie gespeist, wobei die Batterie 11 zu diesem Zweck über die Elektrodenleitung 2 mit der. Einrichtung 10 elektrisch verbunden ist. Gleichermaßen könnte ein aktiver Sensor durch die Batterie 11 mit elektrischer Energie gespeist werden.

Im Sensorsystem 1 können sowohl die Batterie 11 als auch die Diagnoseeinheit 10 jeweils unabhängig voneinander von der Elektrodenleitung 2 entfernt werden. So kann die Batterie 11 in einfacher Weise nachgeladen oder ausgewechselt werden, was insbesondere ermöglicht, dass die Batterie 11 relativ klein, beispielsweise in Form preiswerter und allgemein verfügbarer Knopfzellen, ausgeführt sein kann. Gleichermaßen kann die Diagnoseeinheit 10 in einfacher Weise gewartet oder ausgetauscht werden, wobei es insbesondere ermöglicht ist, dass der Datenspeicher zur Speicherung von EKG-Signalen oder hierauf basierender Daten von einem externen Lesegerät beispielsweise in periodischen Zeitabständen ausgelesen wird. Gleichermaßen kann durch das Entfernen der Endkappe 9 von der Elektrodenleitung 2 Zugang zu einer wechselbaren Speicherkarte geschaffen werden, um die Speicherkarte zu entfernen und die darauf gespeicherten Daten zu lesen. Anschließend kann dieselbe oder eine andere Speicherkarte in die Endkappe 9 eingesetzt werden. Grundsätzlich wäre aber auch denkbar, dass die Endkappe 9 so ausgebildet ist, dass eine wechselbare Speicherkarte auch ohne Abnehmen der Endkappe 9 von der Elektrodenleitung 2 von der Endkappe 9 entnommen werden kann.

Optional kann die Endkappe 9 auch über eine mit der Diagnoseeinheit 10 gekoppelte Telemetrieeinheit mit integrierter Telemetrieschnittstelle (nicht gezeigt) zur drahtlosen Übertragung der abgeleiteten EKG-Signale oder hierauf basierender Daten verfügen. Die Telemetrieeinheit kann insbesondere dazu ausgebildet sein, die Daten zu einem tragbaren Patientengerät zu senden, das diese Daten anschließend an ein Service Center zur Auswertung durch medizinisches Personal (z.B. Arzt) weiterleitet. Auf diese Weise kann insbesondere eine Echtzeit-Überwachung des Patienten (Bio-Watch) realisiert werden.

Wie in Fig. 2 und 3 gezeigt, umfasst die Elektrodenleitung 2 weiterhin mehrere Wirkstoffdepots 12 zur Elution von Wirkstoffen, die beispielsweise eine infektionshemmende oder die Epithelialisierung fördernde Wirkung haben können. Die Wirkstoffdepots 12 sind im Zwischenabschnitt 4 beiderseits der beiden elektrischen Pole 16 angeordnet, können aber auch in den beiden Endabschnitten 3, 3' angeordnet sein. Dem Fachmann sind Aufbau und Funktion solcher Wirkstoffdepots 12 wohlbekannt, so dass hier nicht näher darauf eingegangen werden muss.

Für eine Applikation des Sensorsystems 1 wird zunächst die Elektrodenleitung 2 subkutan implantiert, wobei sie durch einen Stichkanal in den Körper eingeführt und an den beiden Austritts- bzw. Perforationsstellen 5 auf der Haut des Patienten 7 mittels der beiden Endkappen 9, 9' fixiert wird. Nach Abschluss der Wundheilung kann angenommen werden, dass der Stichkanal entlang der Elektrodenleitung 2 vollständig epithelialisiert ist, so dass Infektionen unwahrscheinlich sind. Dies gilt insbesondere dann, wenn die Epithelialisierung fördernde Wirkstoffe von den Wirkstoffdepots 12 abgegeben werden. Die Elektrodenleitung 2 ist hier beispielsweise isodiametrisch ausgebildet, d.h. sie verfügt über einen konstanten Durchmesser, und weist keine Durchmessersprünge auf. Dies ermöglicht, dass die Elektrodenleitung 2 nach Abschluss der Wundheilung (ca. 4 bis 8 Wochen) temporär entfernt und anschließend wieder in den vorhandenen Hautkanal eingesetzt werden kann. Dies kann durch medizinisches Personal, aber auch durch den Patienten 7 selbst erfolgen, da bei dieser Prozedur keine neuerliche Verletzung der Körperoberfläche 6 entsteht.

Das Sensorsystem 1 bildet somit einen EKG-Rekorder dar, bei welchem lediglich die Elektrodenleitung 2 subkutan implantiert ist.

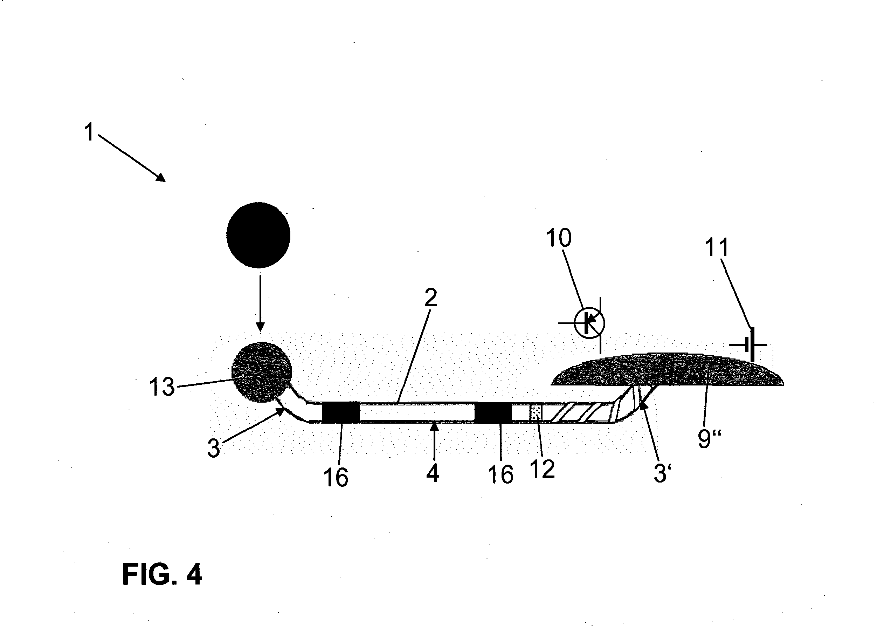

In Fig. 4 ist ein weiteres Sensorsystem 1 veranschaulicht. Um unnötige Wiederholungen zu vermeiden, werden lediglich die Unterschiede zu dem in Verbindung mit den Figuren 1 bis 3 gezeigten Sensorsystem 1 erläutert und ansonsten wird auf die dort gemachten Ausführungen Bezug genommen. Demnach umfasst das Sensorsystem 1 eine Elektrodenleitung 2, die an einem Endabschnitt 3' mit einer Endkappe 9" permanent verbunden ist. In die Endkappe 9" ist sowohl die Diagnoseeinheit 10 (gegebenenfalls gekoppelt mit einer Telemetrieeinheit) als auch die Batterie 11 integriert. Am anderen Endabschnitt 3 ist die Elektrodeleitung 2 mit einem hier beispielsweise in Form einer Kugel ausgebildeten Endstück 13 beispielsweise durch eine Verschraubung verbindbar. Das Endstück 13 dient lediglich für eine Lagefixierung der im Körper des Patienten 7 implantierten Elektrodenleitung 2. Für eine Applikation, wird die mit der Endkappe 9" verbundene Elektrodenleitung 2 durch eine Perforationsstelle 5 implantiert, wo lediglich das Endstück 13 zur Lagefixierung am freien Endabschnitt 3 der Elektrodenleitung 2 zu befestigen ist.

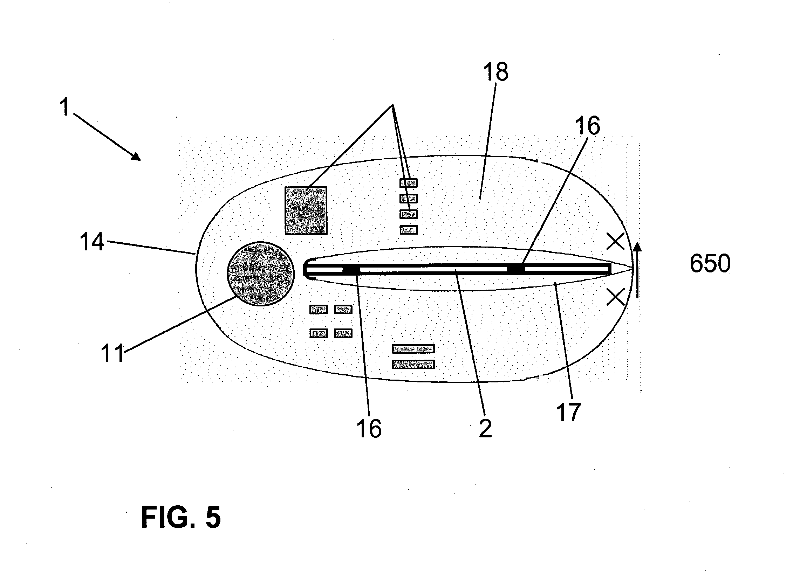

In Fig. 5 bis 7 ist ein Ausführungsbeispiel des erfindungsgemäßen Sensorsystems 1 veranschaulicht. Um unnötige Wiederholungen zu vermeiden, werden wiederum lediglich die Unterschiede zum Ausführungsbeispiel der Figuren 1 bis 3 erläutert und ansonsten auf die dort gemachten Ausführungen Bezug genommen. Demnach umfasst das Sensorsystem 1 ein Substrat bzw. Träger 14 aus einem flexiblen Material mit einer hier beispielsweise zumindest annähernd ovalen Kontur. Der Träger 14 besteht vorzugsweise aus einem biokompatiblen Werkstoff, insbesondere ein Polymerwerkstoff, beispielsweise ein Flüssigkristallpolymer (LCP = Liquid Crystal Polymer). Am Träger 14 sind sowohl die elektronischen Bauteile 15 der Diagnoseeinheit 10 als auch die Batterie 11 befestigt oder darin integriert. Vorzugsweise besteht der Träger 14 aus einem für gedruckte Schaltungen geeigneten Material, so dass die Verdrahtung für die Batterie 11 und die elektronischen Bauteile 15 in einfacher Weise auf den Träger 14 gedruckt werden können. Zum Schutz vor Umwelteinflüssen können die verschiedenen elektronischen bzw. elektrischen Komponenten mit einer Deckschicht oder Kappe (nicht gezeigt) abgedeckt werden. Weiterhin ist eine versteifte Struktur als nadelförmige Elektrodenleitung 2 aus dem Träger 14 geformt. Die Elektrodenleitung 2 befindet sich bis auf einen Endabschnitt, an dem sie mit dem übrigen Trägerabschnitt 18 verbunden ist, im Bereich eines Freischnitts 18. Die Elektrodenleitung 2 ist mit zwei elektrisch leitenden Polen 16 zur Ableitung der EKG-Signale versehen.

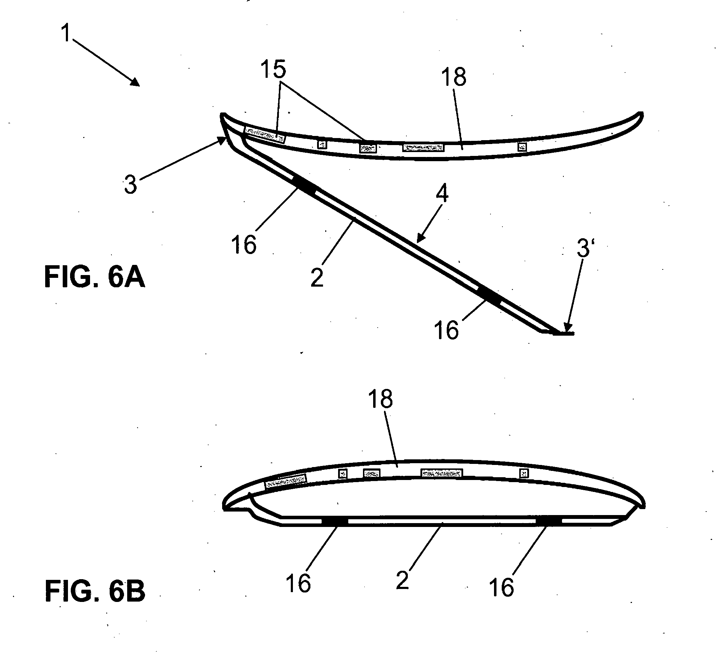

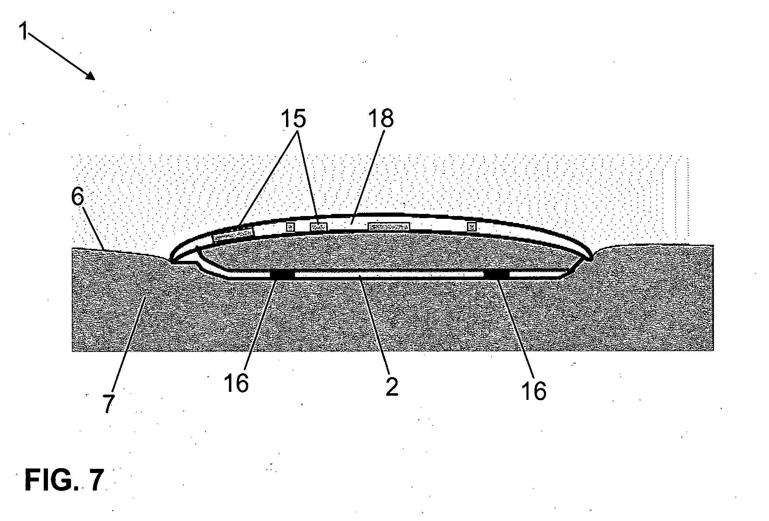

Der Träger 14 kann an den in Fig. 5 mit "X" gekennzeichneten Stellen übereinander gelegt und miteinander verbunden, beispielsweise vernietet, werden, wodurch das Sensorsystem 1 eine clipartige Struktur enthält, in der die Elektrodenleitung 2 relativ zum übrigen Trägerabschnitt 18 aufgrund der Flexibilität des Trägermaterials bewegt werden kann. Fig. 6A zeigt einen ersten bzw. geöffneten Zustand mit abgespreizter Elektrodenleitung 2, Fig. 6B einen zweiten bzw. geschlossenen Zustand mit zurückgeklappter Elektrodenleitung 2. Im geöffneten Zustand kann die nadelförmige Elektrodenleitung 2 in einen subkutanen Kanal eingeführt werden, im geschlossenen Zustand wird die implantierte Elektrodenleitung 2 fixiert. Wie in Fig. 7 gezeigt, befindet sich nur die nadelförmige Elektrodenleitung 2 unter der Haut und der übrige Trägerabschnitt 18 liegt zur Fixierung der Elektrodenleitung 2 der Hautoberfläche auf.

Wie anhand des Ausführungsbeispiels ausführlich erläutert ist, ermöglicht die vorliegende Erfindung eine dauerhafte Implantierung einer Sensorleitung mit einem oder mehreren Sensoren im Körper eines Patienten ("diagnostisches Piercing"), wobei die Implantation sehr einfach und mit stark verringertem Komplikationsrisiko durchgeführt werden kann. Die Sensorleitung kann sehr einfach und insbesondere temporär wieder entfernt werden. Insbesondere können im Sensorsystem preiswerte und austauschbare Komponenten zur Energieversorgung eingesetzt werden, so dass der implantierte Teil des Sensorsystems stark verkleinert werden kann. Dabei kann die Sensorleitung kosmetisch ansprechend gestaltet werden, wobei nach der Explantation keine oder nur sehr kleine Narben verbleiben. Zudem kann auf eine Telemetriefunktion verzichtet werden und stattdessen ein insbesondere wechselbares (Standard-)Speichemedium für Daten, insbesondere eine Speicherkarte, eingesetzt werden, die durch ein externes Lesegerät ausgelesen werden kann.

### Bezugszeichenliste

- 1: Sensorsystem
- 2: Elektrodenleitung
- 3,3': Endabschnitt
- 4: Zwischenabschnitt
- 5: Perforationsstelle
- 6: Körperoberfläche
- 7: Patient
- 8: Verschlusseinrichtung
- 9, 9', 9": Endkappe
- 10: Diagnoseeinheit
- 11: Batterie
- 12: Wirkstoffdepot
- 13: Endstück
- 14: Träger
- 15: elektronisches Bauteil
- 16: Pol
- 17: Freischnitt
- 18: Trägerabschnitt
- 100: EKG-Rekorder
- 101: Patient
- 102: Implantattasche
- 103: Daten

## Patentansprüche

1. Medizinisches Sensorsystem (1), welches umfasst:
- mindestens eine im menschlichen oder tierischen Körper implantierbare Sensorleitung (2) mit zwei Endabschnitten (3, 3'), die dazu ausgebildet sind, durch zwei künstliche Körperöffnungen (5) an die Körperoberfläche (6) geführt zu werden, wobei die Sensorleitung (2) zwischen den beiden Endabschnitten (3) über wenigstens einen Sensor (16) zum Erfassen einer biologischen Messgröße verfügt,
- wenigstens ein auf der Körperoberfläche (6) angeordnetes Fixierelement (9, 9', 9"; 13; 18), welches mit den durch die Körperöffnungen (5) geführten Endabschnitten (3, 3') zur Fixierung der implantierten Sensorleitung (2) verbindbar ist, wobei das wenigstens eine Fixierelement (9, 9', 9"; 13; 18) eine Einrichtung (10) zum Prozessieren von Sensorsignalen sowie eine Energiequelle (11) zur Versorgung des Sensorsystems mit elektrischer Energie aufweist,
wobei es ein einziges Fixierelement (18) zur Fixierung der implantierten Sensorleitung (2) umfasst und das Fixierelement (18) mit einem der beiden Endabschnitte (3) so verbunden ist, dass es relativ zur Sensorleitung (2) bewegbar ist, und mit dem anderen Endabschnitt (3') verbindbar ist, und
**dadurch gekennzeichnet, dass**
es über einen Träger (14) aus einem flexiblen Material verfügt, wobei die Einrichtung (10) zum Prozessieren von Sensorsignalen und die Energiequelle (11) zur Versorgung des Sensorsystems mit elektrischer Energie am Träger (14) befestigt sind und wobei die Sensorleitung (2) in Form einer versteiften Struktur des Trägers (14) ausgebildet ist.

2. Sensorsystem (1) nach Anspruch 1, **dadurch gekennzeichnet, dass** das wenigstens eine Fixierelement (9, 9', 9"; 13; 18) zur Anpassung an die Kontur der Körperoberfläche (6) flexibel ausgebildet ist.

3. Sensorsystem (1) nach einem der Ansprüche 1 oder 2, **dadurch gekennzeichnet, dass** das wenigstens eine Fixierelement (9, 9', 9"; 13; 18) mit den beiden Endabschnitten (3, 3') der Sensorleitung abnehmbar verbindbar ist.

4. Sensorsystem (1) nach einem der Ansprüche 1 bis 3, **dadurch gekennzeichnet, dass** die Sensorleitung (2) flexibel ausgebildet ist.

5. Sensorsystem (1) nach einem der Ansprüche 1 bis 4, **dadurch gekennzeichnet, dass** die Sensorleitung (2) einen konstanten Leitungsdurchmesser aufweist und/oder keine sprunghaften Durchmesseränderungen hat.

6. Sensorsystem (1) nach einem der Ansprüche 1 bis 5, **dadurch gekennzeichnet, dass** die Einrichtung (10) zum Prozessieren von Signalen des Sensors mit einer Telemetrieeinrichtung mit Telemetrieschnittstelle zur Übertragung von Sensorsignalen und/oder hierauf basierender Daten gekoppelt ist.

7. Sensorsystem (1) nach einem der Ansprüche 1 bis 6, **dadurch gekennzeichnet, dass** die Sensorleitung (2) mindestens ein Wirkstoffdepot (12) zur Abgabe eines oder mehrerer Wirkstoffe an den Körper aufweist.

8. Sensorsystem (1) nach einem der Ansprüche 1 bis 7, **dadurch gekennzeichnet, dass** es eine Sicherungseinrichtung zum Verhindern eines Lösens der Verbindung zwischen dem mindestens einen Fixierelement (9, 9', 9"; 13; 18) und den beiden Endabschnitten (3, 3') der Sensorleitung (2) aufweist.

9. Sensorsystem (1) nach einem der Ansprüche 1 bis 8, **dadurch gekennzeichnet, dass** die Sensorleitung (2) in Form einer bipolaren Elektrodenleitung mit zumindest einer Elektrode (16) zum Erfassen elektrischer Signale ausgebildet ist.

## Claims

1. A medical sensor system (1), which comprises:
- at least one sensor lead (2), which can be implanted in a human or animal body and has two end portions (3, 3') configured to be routed through two artificial body openings (5) on the body surface (6), wherein the sensor lead (2), between the two end portions (3), has at least one sensor (16) for detecting a biological measured variable,
- at least one fixing element (9, 9', 9"; 13; 18), which is disposed on the body surface (6) and which can be connected to the end portions (3, 3') routed through the body openings (5) in order to fix the implanted sensor lead (2), wherein the at least one fixing element (9, 9', 9"; 13; 18) has a device (10) for processing sensor signals and an energy source (11) for supplying the sensor system with electrical energy,
wherein said medical sensor system comprises a single fixing element (18) for fixing the implanted sensor lead (2), and the fixing element (18) is connected to one of the two end portions (3) such that it can be moved relative to the sensor lead (2) and can be connected to the other end portion (3'), and
**characterised in that**
said medical sensor system has a carrier (14) made of a flexible material, wherein the device (10) for processing sensor signals and the energy source (11) for supplying the sensor system with electrical energy are fastened to the carrier (14), and wherein the sensor lead (2) is formed as a reinforced structure of the carrier (14).

2. The sensor system (1) according to claim 1, **characterised in that** the at least one fixing element (9, 9', 9"; 13; 18) is flexible in order to enable adaptation to the contour of the body surface (6).

3. The sensor system (1) according to either of claims 1 and 2, **characterised in that** the at least one fixing element (9, 9', 9"; 13; 18) is detachably connectable to the two end portions (3, 3') of the sensor lead.

4. The sensor system (1) according to one of claims 1 to 3, **characterised in that** the sensor lead (2) is flexible.

5. The sensor system (1) according to one of claims 1 to 4, **characterised in that** the sensor lead (2) has a constant lead diameter and/or does not have any abrupt changes in diameter.

6. The sensor system (1) as claimed in one of claims 1 to 5, **characterised in that** the device (10) for processing signals of the sensor is coupled to a telemetry device having a telemetry interface for the transmission of sensor signals and/or data based hereon.

7. The sensor system (1) according to one of claims 1 to 6, **characterised in that** the sensor lead (2) has at least one active ingredient reservoir (12) for delivering one or more active ingredients to the body.

8. The sensor system (1) according to one of claims 1 to 7, **characterised in that** it has a securing arrangement for preventing a detachment of the connection between the at least one fixing element (9, 9', 9"; 13; 18) and the two end portions (3, 3') of the sensor lead (2).

9. The sensor system (1) according to one of claims 1 to 8, **characterised in that** the sensor lead (2) is formed as a bipolar electrode lead having at least one electrode (16) for detecting electrical signals.

## Revendications

1. Système de capteur médical (1), lequel comprend :
- au moins une ligne de capteur (2) implantable dans le corps humain ou animal, avec deux parties d'extrémité (3, 3') qui sont conçues pour être passées par deux orifices artificiels (5) à la surface du corps (6), où la ligne de capteur (2) dispose d'au moins un capteur (16) entre les deux parties d'extrémité (3) pour la détermination d'une valeur de mesure biologique,
- au moins un élément de fixation (9, 9', 9" ; 13 ; 18) disposé sur la surface du corps (6), lequel peut être relié avec les parties d'extrémité (3, 3') traversant les orifices du corps (5) pour la fixation de la ligne de capteur (2) implantée, où l'au moins un élément de fixation (9, 9', 9" ; 13 ; 18) présente un équipement (10) pour la mise en oeuvre de signaux de capteur ainsi qu'une source d'énergie (11) pour l'alimentation du système de capteur avec de l'énergie électrique,
où il comprend un seul élément de fixation (18) pour la fixation de la ligne de capteur (2) implantée et l'élément de fixation (18) est relié avec l'une des deux parties d'extrémité (3) de telle manière qu'il est mobile par rapport à la ligne de capteur (2), et peut être relié avec l'autre partie d'extrémité (3'), et
**caractérisé en ce**
**qu'**il dispose d'un support (14) constitué d'un matériau souple, où l'équipement (10), pour le traitement de signaux de capteur et la source d'énergie (11), pour une alimentation du système de capteur avec de l'énergie électrique, sont fixés sur le support (14) et où la ligne de capteur (2) est conçue sous la forme d'une structure rigidifiée du support (14).

2. Système capteur (1) selon la revendication 1, **caractérisé en ce que** l'au moins un élément de fixation (9, 9', 9" ; 13 ; 18) est conçu de manière souple pour un ajustement au contour de la surface du corps (6).

3. Système capteur (1) selon l'une des revendications 1 ou 2, **caractérisé en ce que** l'au moins un élément de fixation (9, 9', 9" ; 13 ; 18) peut être relié avec les deux parties d'extrémité (3, 3') de la ligne de capteur en pouvant en être enlevé.

4. Système capteur (1) selon l'une des revendications 1 à 3, **caractérisé en ce que** la ligne de capteur (2) est conçue de manière souple.

5. Système capteur (1) selon l'une des revendications 1 à 4, **caractérisé en ce que** la ligne de capteur (2) présente un diamètre de ligne constant et/ou n'a aucune modification de diamètre abrupte.

6. Système capteur (1) selon l'une des revendications 1 à 5, **caractérisé en ce que** l'équipement (10) pour le traitement de signaux du capteur est couplé avec un équipement de télémétrie avec une interface télémétrique pour la transmission de signaux de capteur et/ou de données se basant sur ceux-ci.

7. Système capteur (1) selon l'une des revendications 1 à 6, **caractérisé en ce que** la ligne de capteur (2) présente au moins un dépôt de matière active (12) pour l'administration d'une ou de plusieurs matières actives au corps.

8. Système capteur (1) selon l'une des revendications 1 à 7, **caractérisé en ce qu'**il présente un équipement de sécurité pour empêcher un détachement de la liaison entre l'au moins un élément de fixation (9, 9', 9" ; 13 ; 18) et les deux parties d'extrémité (3, 3') de la ligne de capteur (2).

9. Système capteur (1) selon l'une des revendications 1 à 8, **caractérisé en ce que** la ligne de capteur (2) est conçue sous la forme d'une ligne d'électrode bipolaire avec au moins une électrode (16) pour la saisie de signaux électriques.
